(19) Europäisches Patentamt
European Patent Office
Office européen des brevets

(11) Publication number: **0 460 448 B1**

# (12) EUROPEAN PATENT SPECIFICATION

(45) Date of publication of patent specification: **04.10.95**

(51) Int. Cl.$^{6}$: **A61K 38/00**

(21) Application number: **91108165.1**

(22) Date of filing: **21.05.91**

## (54) Use of C63-amide derivatives of 34-de(acetylglucosaminyl)-34-deoxy-teicoplanin against bacteria resistant to glycopeptide antibiotics.

(30) Priority: **28.05.90 EP 90110102**

(43) Date of publication of application:
**11.12.91 Bulletin 91/50**

(45) Publication of the grant of the patent:
**04.10.95 Bulletin 95/40**

(84) Designated Contracting States:
**AT BE CH DE DK ES FR GB GR IT LI LU NL SE**

(56) References cited:
**EP-A- 0 376 041**
**WO-A-88/06600**

(73) Proprietor: **GRUPPO LEPETIT S.p.A.**
**Via Roberto Lepetit, 8**
**I-20020 Lainate (MI) (IT)**

(72) Inventor: **Malabarba, Adriano**
**5/A, Via Roma**
**I-20082 Binasco (MI) (IT)**
Inventor: **Kettenring, Jürgen Kurt**
**27, Via Oronco**
**I-21100 Varese (IT)**

(74) Representative: **Macchetta, Francesco et al**
**GRUPPO LEPETIT S.p.A.**
**Patent and Trademark Department**
**Via Roberto Lepetit, 34**
**I-21040 Gerenzano (Varese) (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 460 448 B1

## Description

This invention is directed to the use of $C^{63}$-amide derivatives of 34-de(acetylglucosaminyl)-34-deoxy-teicoplanins of the formula I

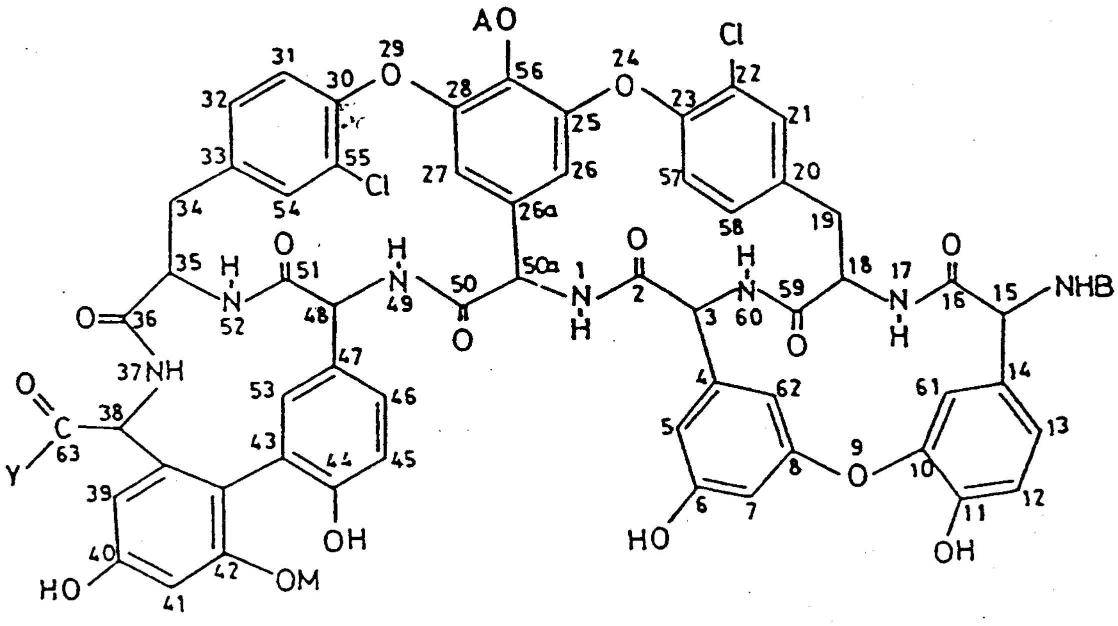

I

wherein:

A represents N[($C_9$-$C_{12}$)aliphatic acyl]-beta-D-2-deoxy-2-aminoglucopyranosyl;
B is hydrogen or a protecting group of the amine function;
M represents alpha-D-mannopyranosyl;
Y represents a di- or poly-amine group of the formula

$-NR-[(CH_2)_mNR^1]_n-X-[(CH_2)_kNR^2]_h-(CH_2)_p-NR^3R^4$

wherein:

R is hydrogen or linear or branched ($C_1$-$C_8$)alkyl;
$R^1$ is hydrogen or linear or branched ($C_1$-$C_8$)alkyl;
$R^2$ is hydrogen or linear or branched ($C_1$-$C_8$)alkyl;
$R^3$ and $R^4$ are each independently hydrogen, linear or branched ($C_1$-$C_8$)alkyl optionally bearing a $NH_2$, OH or SH substituent or taken together with the adjacent nitrogen atom, form a 5 to 7 membered saturated heterocyclic ring which may contain a further heteroatom selected from -S-, -O- and $-NR^5$-wherein $R^5$ is hydrogen, ($C_1$-$C_4$)alkyl, phenyl, or phenyl-($C_1$-$C_4$)alkyl
m, k and p each independently represent an integer from 2 to 8;
n and h, each independently, represent an integer from 0 to 4;
X represents a single bond, or when n is 1, taken together with the adjacent group $NR^1$, it may represent a bifunctional radical of the formula:

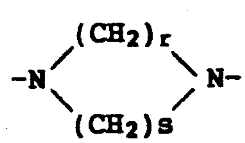 or 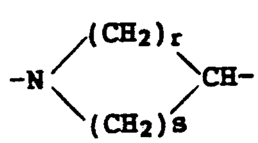

wherein r and s each independently represent an integer from 1 to 6 with the proviso that their sum is an integer from 3 to 8;
and their addition salts with pharmaceutically acceptable acids, for the manufacture of a medicament for

combatting infections caused by bacteria resistant to glycopeptide antibiotics of the vancomycin, ristocetin type including teicoplanin.

According to a preferred embodiment of this invention, the ($C_9$-$C_{12}$) aliphatic acyl radicals of the symbol A preferably are fully saturated or have one unsaturation. Most preferably, they are the following radicals: (Z)-4-decenoyl, 8-methylnonanoyl, decanoyl, 8-methyldecanoyl, 9-methyldecanoyl, 6-methyloctanoyl, nonanoyl, 10-methylundecanoyl and dodecanoyl.

The symbols R, $R^1$ and $R^2$, preferably represent hydrogen or linear or branched alkyl radicals of 1 to 4 carbon atoms.

The symbols $R^3$ and $R^4$ each independently preferably represents hydrogen, linear or branched alkyl radicals of 1 to 4 carbon atoms optionally bearing a $NH_2$, OH or SH substituent or $R^3$ and $R^4$ taken together with the adjacent nitrogen atom form a 5 to 7 membered saturated heterocyclic ring which may contain a further heteroatom selected from -S-, -O- and $-NR^5-$, the following heterocyclic rings being the most preferred ones: pyrrolidine, piperidine, oxazolidine, thiazolidine, isoxazolidine, isothiazolidine, morpholine, piperazine, thiomorpholine, hexahydroazepine, hexahydro-1,5- diazepine and hexahydro-1,4-diazepine; $R^5$ preferably is hydrogen or $C_1$-$C_4$ alkyl.

The symbols m, k and p preferably represent integers from 2 to 6, most preferably, from 2 to 4.

The symbols n and h preferably represent 0, 1 or 2, most preferably 0 or 1.

The symbol X preferably represents a single bond or, when n is 1, taken together with the adjacent group $NR^1$ represents a bifunctional radical of the formula

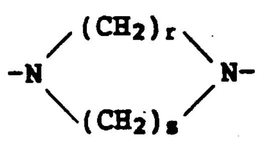

or

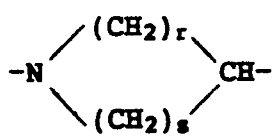

wherein r and s are both 2 or one is 1 and the other is 2 or 3.

According to the general definitions given above representative examples of the group:

$-NR-[(CH_2)_mNR^1]_n-X-[(CH_2)_kNR^2]_h-(CH_2)_p-NR^3R^4$

are the following:

$-NH(CH_2)_2NH_2$;
$-NH(CH_2)_3N(CH_3)_2$;
$-NCH_3(CH_2)_3N(CH_3)_2$;
$-NC_2H_5(CH_2)_3N(n\text{-}C_4H_9)_2$;
$-NH(CH_2)_3NH(n\text{-}C_8H_{17})$;
$-NCH_3(CH_2)_3NHCH_3$;

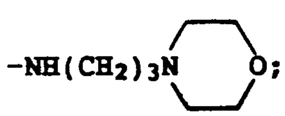 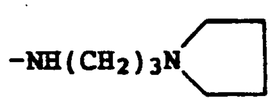 ;

$-NH(CH_2)_3NH(CH_2)_2OH$; $-NH(CH_2)_2NH(CH_2)_4SH$;
$-NCH_3(CH_2)_4-NC_2H_5(CH_2)_2NHC_2H_5$;
$-NH(CH_2)_4NH_2$;
$-NCH_3(CH_2)_6N(CH_3)_2$;
$-NC_2H_5(CH_2)_5NH_2$;

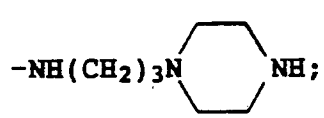

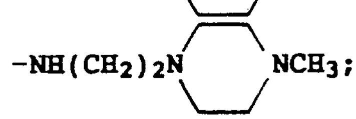

$-NH(CH_2)_2NH(CH_2)_2NH_2$;
$-NH(CH_2)_3NH(CH_2)_3NH_2$;
$-NH(CH_2)_3N[(CH_2)_3NH_2]_2$;
$-NH(CH_2)_3N[(CH_2)_3OH]_2$;
$-NH(CH_2)_3NH(CH_2)_4NH_2$;
$-NH(CH_2)_4NH(CH_2)_3NH_2$;
$-NH(CH_2)_3NH(CH_2)_2NH(CH_2)_3NH_2$;
$-NH(CH_2)_3NH(CH_2)_3NH(CH_2)_3NH_2$;
$-NH(CH_2)_3NH(CH_2)_4NH(CH_2)_3NH_2$;

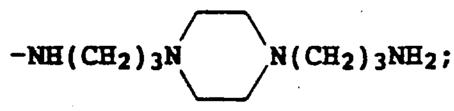

$-NH(CH_2CH_2NH)_2CH_2CH_2NH_2$;
$-NH(CH_2CH_2CH_2NH)_3CH_2CH_2CH_2NH_2$;
$-NCH_3(CH_2)_2NH(CH_2)_3N(CH_3)_2$;
$-NCH_3(CH_2)_3NCH_3(CH_2)_3N(CH_3)_2$;
$-NCH_3(CH_2)_3NH(CH_2)_4N(n\text{-}C_4H_9)_2$;
$-NH(CH_2)_3NH(CH_2)_4NH(n\text{-}C_8H_{17})$;
$-NH(CH_2)_3NH(CH_2)_4NH(CH_2)_3N(n\text{-}C_4H_9)_2$;
$-NH(CH_2)_3NH(CH_2)_4NH(CH_2)_3NH(n\text{-}C_8H_{17})$;
$-NCH_3(CH_2)_3NCH_3(CH_2)_3NCH_3(CH_2)_3NHCH_3$;
$-NCH_3(CH_2)_3NCH_3(CH_2)_3NCH_3(CH_2)_3N(n\text{-}C_4H_9)_2$;

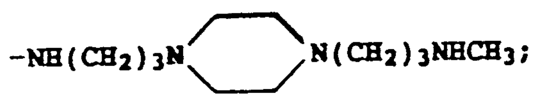

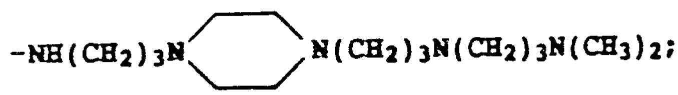

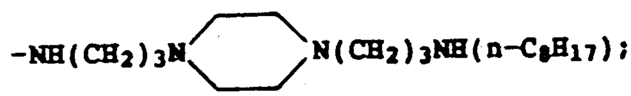

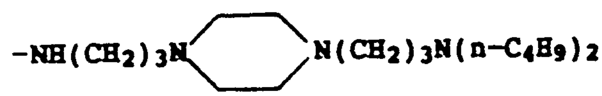

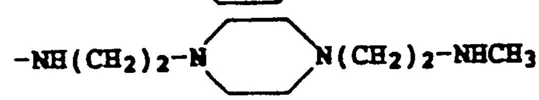

The compounds above described show antimicrobial activity, in particular, against gram-positive bacteria, including Group A Streptococci and some coagulase-negative Staphylococci. They are described in the European Patent Application Publication No. 376041 in the name of Gruppo Lepetit S.p.A.

Various $C^{63}$-amide derivatives of teicoplanin complex, single components and the aglycone and pseudo aglycones thereof are described in European Patent Application Publication No. 218099 and International Patent Application Publication No. WO 88/06600.

The compounds reported in this application are prepared by amidation of the corresponding 34-de-(acetylglucosaminyl)-34-deoxy-teicoplanin derivatives of formula I wherein Y is OH, (i.e. the corresponding carboxy acids). These starting materials are specifically described in the European Patent Application Publication No. 290922 or can be prepared according to the procedure disclosed therein.

The above mentioned starting materials are prepared either from the teicoplanin $A_2$ complex (as resulting from fermentation operations) or from its five main components (see: U.S. Patent 4,542,018; A. Borghi et al., J. Antibiot. Vol. 37, 615-620, 1984; C.J. Barna et al. J. Am. Chem. Soc. 1984, 106, 4895-4902) by elimination of the acetylglucosaminyl rest at the position 34.

As it is known in the art, the above mentioned five main components of teicoplanin $A_2$ complex are characterized by the fact that the aliphatic acyl moiety of the beta-D-2-deoxy-2-aminoglucopyranosyl rest is a ($C_{10}$-$C_{11}$) aliphatic acyl, namely: (Z)-4-decenoyl, 8-methylnonanoyl, decanoyl, 8-methyldecanoyl or 9-methyldecanoyl. Accordingly, the resulting substances used as starting material for the manufacture of the compounds of this invention can be either individual products or mixtures of one or more products. Since said starting materials for the preparation of the compounds of this invention can be used in both said forms, the resulting end products may, in turn, be individual compounds or mixtures of two or more compounds of the above formula I. These mixtures of compounds are also part of the invention and may be used as such for their biological applications and uses or may be eventually separated in their individual components by known procedures described in the art. Examples of separation procedures suitable for the purpose of obtaining individual components from end products mixtures of teicoplanin amide derivatives are those described in the following documents: European Patent Application Publication No. 218099 and International Patent Application Publication No. WO 88/06600.

Other starting materials for the preparation of the compounds of this invention can be obtained by applying the process of the European Patent Application Publication No. 290922 to teicoplanin compounds such as those described as compound B (identified also as "RS-4" in the papers mentioned below) and compound A (identified also as "RS-3" in the papers mentioned below) in the European Patent Application Publication No. 306645, and those identified as teicoplanin compounds RS-1 and RS-2 in the paper given by M. Zanol et al. at the 17th International Symposium on Chromatography, Vienna, September 25-30, 1988 (see also A. Borghi et al. The Journal of Antibiotics, Vol. 42, No. 3, 361-366, 1989). Said teicoplanin compounds are characterized by the fact that the aliphatic acyl moieties of the beta-D-2-deoxy-2-amino-glucopyranosyl rest are respectively: nonanoyl, 6-methyloctanoyl, 10-methylundecanoyl and dodecanoyl.

The amidation procedures described in the above mentioned European Patent Application Publication No. 218099 and International Patent Application Publication No. WO 88/06600 can be used also for the preparation of the compounds described in the present application. Said procedures involve condensing the carboxy acid starting materials mentioned above with on excess of the appropriate amine of the formula II:

$NHR\text{-}[(CH_2)_mNR^1]_n\text{-}X\text{-}[(CH_2)_kNR^2]_h\text{-}(CH_2)_p\text{-}NR^3R^4$ II

wherein R, $R^1$, $R^2$, $R^3$, $R^4$, X, m, n, h, k and p have the same meanings as above, in an inert organic solvent in the presence of a condensing agent selected from ($C_1$-$C_4$)alkyl, phenyl or heterocyclyl phosphorazidates at a temperature between 0°C and 20°C. If the amine reactant contains other functions which are not inert under the selected reaction conditions, said functions are suitably protected by means of per se known protecting groups.

According to a preferred embodiment, the compounds of formula I wherein Y is a di-or polyamine group as defined above are prepared by reacting an "activated ester" of the carboxylic acid of the same formula I, wherein Y is OH and the $N^{15}$ -amino function is preferably protected, with the appropriate amine II.

The $N^{15}$-amino function can be protected by methods known per se in the art such as those described in reference books like T.W. Greene, "Protective Groups in Organic Synthesis", John Wiley and Sons, New York, 1981, and M. Mc. Omie "Protecting Groups in Organic Chemistry", Plenum Press, New York, 1973. These protecting groups must be stable at the conditions of the reaction process, must not unfavorably interfere with the amidation reaction, and must be easily cleavable and removable from the reaction medium at the end of the reaction without altering the newly formed wide bond.

Representative examples of N-protecting groups which may be advantageously used for protecting the $N^{15}$ primary amino function of the teicoplanin starting material and, when appropriate, the $NR^3R^4$ moiety of the amine II reactant, are carbamate forming reagents characterized by the following oxycarbonyl groups: 1,1-dimethylpropynyloxycarbonyl, t-butyloxycarbonyl, vinyloxycarbonyl, aryloxycarbonyl, cinnamyloxycarbonyl, benzyloxycarbonyl, p-nitrobenzyloxycarbonyl, 3,4-dimethoxy-6-nitrobenzyloxycarbonyl, 2,4-dichlorobenzyloxycarbonyl, 5-benzisoxazolylmethyloxycarbonyl, 9-anthranylmethyloxycarbonyl, diphenylmethyloxycarbonyl, isonicotinyloxycarbonyl, S-benzyloxycarbonyl, 2,2,2-trichloroethoxycarbonyl, 2,2,2-trichloro-t-butoxycarbonyl, and the like. Other suitable N-protecting agents are aldehydes or ketones, or derivatives thereof which are capable of forming Schiff bases with the amino group to be protected.

Preferred examples of such Schiff base forming agents are benzaldehydes and particularly preferred is 2-hydroxybenzaldehyde (salicylaldehyde). A convenient means of protection in the case the amine II reactant has R different from hydrogen and contains a primary amino function (e.g. $R^3$ and $R^4$ both represent hydrogen) is, in some instances, the formation of a benzyliden derivative which may be prepared by reacting the mine with benzaldehyde in a lower alkanol, such as ethanol, preferably at room temperature. After the reaction with the selected teicoplanin starting material has been completed, the benzylidene

protecting group may be removed as known in the art, e.g. by catalytic hydrogenation, using, for instance, Palladium on carbon as the catalyst.

However, in all cases where catalytic hydrogenation is applied, attention should be paid to the presence of groups which may be modified by catalytic hydrogenation. A typical consequence of the catalytic hydrogenation of an amino-protected derivative of formula I wherein A represents a group as above defined whose acyl portion is (Z)-4-decenoyl (or a mixture containing it) is that, at least partially, the decenoyl compound is transformed into the corresponding decanoyl compound. Therefore, when the removal of the protecting group is carried out through catalytic hydrogenation and the 34-de(acetylglucosaminyl)-34-deoxy-teicoplanin starting material is (or contains) a derivative of component 1 of teicoplanin $A_2$ complex (whose acyl portion is (Z)-4-decenoyl) the final amide product, in most cases, does not contain the corresponding derivative but, rather, a proportionally larger amount of the derivative of the component 3 whose acyl rest is decanoyl.

If a final compound containing the amide derivative of teicoplanin $A_2$ complex component 1 is desired, the N-protecting group must be selected among those which can be removed under conditions which do not imply hydrogenation of the acyl portion or hydrolysis of the sugar moieties of the teicoplanin substrate. For example, an N-protecting group which is removable under mild conditions is selected from beta-halo-alkoxycarbonyl group, such as 2,2,2-trichloro-tert-butoxycarbonyl, which can be removed according to the procedures described by H. Eckert et al., in Angew. Chem. Int. Ed. Engl. 17, No.5, 361-362 (1978).

As it is appreciated by the skilled technician, the ultimate choice of the specific protecting group depends on the characteristics of the particular amide derivative which is desired. In fact, this amide function of the final compound should be stable at the condition of removal of the protecting group(s).

Since the conditions of removal of the different protecting groups are known, the skilled technician is capable of selecting the proper protecting group.

In some cases, when the amine II contains two primary amino groups (e.g. R, $R^3$ and $R^4$ all representing hydrogen) it may be convenient to obtain a mixture of two reaction products resulting from the formation of the amidic bond with each of the two primary aminic functions and to separate them by common procedures such as flash column chromathography, reverse-phase column chromathography or preparative HPLC.

The formation of "activated esters" is described in general terms in Fieser and Fieser, "Reagents for Organic Synthesis", John Wiley and Sons Inc. 1967 pages 129-130.

Examples of said activated ester forming reagents that can be conveniently used in the process of the invention are those described by R. Schwyzer et al. in Helv. Chim. Acta, 1955, 38, 69-70 and encompass: $ClCH_2CN$, $BrCH_2COOC_2H_5$, $BrCH(COOC_2H_5)_2$, $ClCH_2COCH_3$,

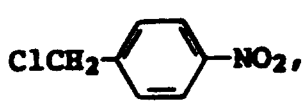, $ClCH_2CH_2N(C_2H_5)_2$

A preferred reagent of this type is chloroacetonitrile. In this case, chloroacetonitrile itself or dimethylformamide (DMF) can be used as preferred solvents.

Generally, inert organic solvents useful for the formation of "activated esters" are those organic aprotic solvents which do not unfavorably interfere with the reaction course and are capable of, at least partially, solubilizing the carboxy acid starting material.

Examples of said inert organic solvents are organic amides, alkyl ethers, ethers of glycols and polyols, phosphoramides, sulfoxides and aromatic compounds. Preferred examples of inert organic solvents are: dimethylformamide, dimethoxyethane, hexamethylphosphoramide, dimethylsulfoxide, benzene, toluene and mixtures thereof.

More preferably, the solvent is selected from acetonitrile, dimethylsulfoxide, dimethylformamide. The formation of the activated ester is generally conducted in the presence of a base which does not interfere with the reaction course such as a trialkylamine like triethylamine, sodium or potassium carbonate or bicarbonate. Generally, the base is employed in a 2 to 6 molar proportion to the teicoplanin carboxy acid starting material and, preferably, it is used in an about three-fold molar excess. A preferred base is triethylamine.

The "activated ester" forming reagent is used in a large excess over the teicoplanin carboxy acid starting material. It is in general used in a 5 to 35 molar proportion and, preferably, it is used in an about 20 to 30 times molar excess. The reaction temperature is between 10 °C and 60 °C and preferably between

15°C and 30°C. As usual, the reaction time depends on the other specific reaction parameters and may be generally between 3 and 48 hours.

In this case, the reaction course may be followed by HPLC or TLC to determine when the reaction may be considered as completed and the procedures to recover the desired intermediate can be started. The "activated ester" intermediate can be directly used in the same reaction medium where it is prepared, however, in general, it is isolated by precipitation with non-solvents or by extraction with solvents and it is used as such, without further purification, in the next reaction step. If desired, however, it may be purified by column chromatography such as flash column chromatography or reverse-phase column chromatography.

The obtained "activated ester" intermediate is then reacted with a molar excess of the amine derivative of formula II

$NHR-[(CH_2)_mNR^1]_n-X-[(CH_2)_kNR^2]_h-(CH_2)_p-NR^3R^4$

in the presence of an organic polar solvent at a temperature between 5°C and 60°C, preferably between 10°C and 30°C.

The organic polar solvent can be in this case a polar protic or aprotic solvent.

Preferred examples of organic polar protic solvents are lower($C_2$-$C_4$) alkanols such as, ethanol, n-propanol, iso-propanol, n-butanol and the like, or mixtures thereof, preferably used in the dry form.

Preferred examples of organic polar aprotic solvent are N,N-dimethylformamide (DMF), hexamethylphosphoramide (HMPA), or mixtures thereof, 1,3-dimethyl-3,4,5,6-tetrahydro-2(1H)pyrimidone (DMPU), dimethylsulfoxide or dimethoxyethane.

The reaction of the "activated ester" with the selected amine can be carried out at a temperature between 5°C and 60°C but the preferred temperature is generally comprised between 10°C and 30°C, most preferably between 20°C and 25°C, while a preferred molar proportion between the "activated ester" intermediate and the amine II as above defined is from 1:5 to 1:30, and more preferably from 1:10 to 1:20. The reaction course may be monitored as usual by TLC or HPLC.

The amide derivative obtained from the amidation reaction is recovered from the reaction solution according to common procedures, for instance, by evaporation of the solvent or by addition of a non-solvent. The removal of the amino-protecting group is usually carried out on the crude product isolated from the amidation reaction.

Examples of procedures for the removal of said protecting groups from teicoplanin derivatives are described for instance in International Patent Application Publication No. WO 88/06600.

If catalytic hydrogenation procedures are used, the reaction is usually carried out in the presence of a diluted aqueous strong acid, preferably a mineral acid, in an organic solvent miscible with said diluted aqueous strong acid. The filtrate from the reaction is then worked for the recovery of either the mineral acid addition salt of the amide of formula I or the corresponding free base. Analogous procedures are followed when the amino-protecting group is a group which can be removed by treating with diluted mineral acids (e.g. Schiff base or a $C_1$-$C_4$ alkoxy carbonyl group) under conditions which do not cause the splitting of the sugar moieties (e.g. low temperatures, short reaction time).

For the isolation of the acid addition salt, the reaction solution resulting from the splitting of the amino-protecting group is generally brought to a pH value between 6 and 7 by addition of an aqueous base, e.g. aqueous sodium hydroxide, and, after evaporation of the solvent under reduced pressure, the resulting solid is separated in the form of an addition salt with the strong acid which has been added during the deprotection step. Such product may be further purified by common techniques e.g. column chromatography, precipitation from solutions by addition of non-solvents, preparative HPLC and similar. The acid addition salt may be converted to the corresponding free base of formula I by suspending or dissolving the acid addition salt in an aqueous solvent which is then brought to an appropriate pH value whereby the free base form is restored. The product is then recovered, for instance, by extraction with an organic solvent or is transformed into another acid addition salt by adding the selected acid and working up as above.

Sometimes, after the above operation, it may be necessary to submit the recovered product to a common desalting procedure.

For example, column chromatography on controlled pore polydextrane resins (such as Sephadex L H 20) or silanized silica gel may be conveniently used. After eluting the undesired salts with an aqueous solution, the desired product is eluted by means of linear gradient or step-gradient of a mixture of water and a polar or apolar organic solvent, such as acetonitrile/water or acetonitrile/aqueous acetic acid from 5% to about 100% acetonitrile and then recovered by evaporation of the solvent or by lyophilization.

When a compound of formula I is obtained in the free base form, it can be transformed into the corresponding acid addition salt by suspending or dissolving the free base form in an aqueous solvent and adding a slight molar excess of the selected acid. The resulting solution or suspension is then lyophilized to recover the desired acid addition salt. Instead of lyophilizing, in some instances, it is possible to recover the final salt through precipitation by addition of a non-solvent mixable with water.

In case the final silt is unsoluble in an organic solvent where the free base form is soluble it may be recovered by filtration from the organic solution of the non-salt form after addition of the stoichiometric amount or a slight molar excess of the selected acid.

Representative and suitable acid addition salts of the compounds of formula I include those salts formed by standard reaction with both organic and inorganic acids such as, for example, hydrochloric, hydrobromic, sulfuric, phosphoric, acetic, trifluoroacetic, trichloroacetic, succinic, citric, ascorbic, lactic, maleic, fumaric, palmitic, cholic, pamoic, mucic, camphoric, glutaric, glycolic, phthalic, tartaric, lauric, stearic, salicylic, methanesulfonic, benzenesulfonic, sorbic, picric, benzoic, cinnamic and the like acids.

Preferred addition salts of the compounds of this invention are the pharmaceutically acceptable acid addition salts.

With the term "pharmaceutically acceptable acid addition salts" are intended those salts with acids which from biological, manufacturing and formulation standpoint are compatible with the pharmaceutical practice.

Examples of acids suitable for the "pharmaceutically acceptable acid addition salts" include those listed above.

A characteristic of the compounds of this invention which further differentiates them from the corresponding starting compound is the configuration of the amidic bond at the 51, 52 position which is "cis", while the configuration of the same bond in the starting teicoplanin carboxylic acid is "trans". This implies that the conformation of the teicoplanin core of the new compounds is remarkably modified with respect to that of the corresponding starting materials.

A peculiarity of these compounds which constitutes the object of the present invention is their ability to inhibit the growth of bacteria resistant to glycopeptide antibiotics of the vancomycin-ristocetin class including teicoplanin.

As can be seen in Table I the compounds of the present invention show a surprisingly improved activity in comparison with teicoplanin, vancomycin and the corresponding amides of teicoplanin against glycopeptide resistant Enterococci strains.

In particular, the activity of the compounds of the invention was confirmed through in vitro experiments carried out with clinical isolates resistant to glycopeptide antibiotics such as with Enterococci faecalis, Enterococci faecium, as shown in Table I.

Therefore, a further aspect of the invention concerns the use of the present compounds for the treatment of infectious diseases provoked by bacteria which are resistant to glycopeptide antibiotics of the vancomycin and ristocetin type such as teicoplanin and its amide derivatives, in particular by Enterococci strains resistant to glycopeptide antibiotics.

The amide derivatives of teicoplanin which are reported in Table I were described in European Patent Applications Publication Nos. 218099 and 326873, International Patent Application Publication No. WO 90/11300 and in European Patent Application Publication No. 370283.

## TABLE I

In vitro activity (MIC, mcg/ml) of C63-amide derivatives of 34-de(acetylglucosaminyl)-34-deoxy teicoplanin CTI-AMIDES, in comparison with the corresponding teicoplanin amides, teicoplanin (TEICO) and vancomycin (VANCO), against glycopeptide-resistant Enterococci (E.)

| ORGANISM | STRAIN NO. (internal code) | COMPOUNDS OF THE INVENTION | | | | | TEICOPLANIN AMIDES | | | | | TEICO | VANCO |
|---|---|---|---|---|---|---|---|---|---|---|---|---|---|
| | | 1 | 2 | 3 | 4 | 5 | 1' | 2' | 3' | 4' | 5' | | |
| E. faecalis | NCTC 12201 | 32 | 16 | 8 | 32 | 8 | 128 | 128 | 32 | 8 | 32 | 128 | >128 |
| E. faecalis | L 560 | 16 | 16 | 16 | 32 | 16 | 128 | 128 | 64 | 64 | 128 | >128 | >128 |
| E. faecalis | NCTC 12202 | 32 | 32 | 32 | 64 | 16 | >128 | >128 | 128 | 128 | 64 | 128 | >128 |
| E. faecium | NCTC 12203 | 16 | 16* | 8 | 16 | 8 | 128 | 128 | 64 | 32 | 32 | 64 | >128 |
| E. faecium | NTCT 12204 | 16 | 16 | 16 | 16 | 8 | >128 | 128 | 64 | 32 | 16 | 64 | >128 |
| E. faecium | L 1666 | >128 | >128 | 128 | 128 | 64 | >128 | >128 | >128 | >128 | >128 | >128 | >128 |

The antibacterial activity is determined in vitro by means of standard agar-dilution tests in microtiter. Isosensitest broth (Oxoid) is used for growing Enterococci. Broth cultures are diluted so that the final inoculum is about 5 x $10^5$ forming units/ml (CFU/ml).

Minimal inhibitory concentration (MIC) is considered as the lowest concentration which shows no visible growth after 18-24 h incubation at 37°C.

The $ED_{50}$ values (mg/kg) of representative compounds of the invention in vivo tests in mice experimentally infected with S. pyogenes C203 according to the procedure described by V. Arioli et al., Journal of Antibiotics 29, 511 (1976) are reported in Table II below:

TABLE II

| Compound No. | $ED_{50}$ (mg/kg) (s.c.) |
|---|---|
| 3 | 0.18 |
| 4 | 0.12 |
| 5 | 0.08 |
| 32 | 0.06 |
| 33 | 0.04 |

In view of the above reported antimicrobial activity, the compounds of the present invention can be employed as the active ingredient of antimicrobial preparations used in human and veterinary medicine for the prevention and treatment of infections diseases caused by pathogenic bacteria which are susceptible to said active ingredients.

In such treatments, these compounds may be employed as such or in the form of mixtures in any proportion.

The compounds of the present invention can be administered orally, topically or parenterally wherein however, the parenteral administration is preferred. Depending on the route of administration, these compounds can be formulated into various dosage forms. Preparations for oral administration may be in the form of capsules, tablets, liquid solutions or suspensions. As known in the art the capsules and tablets may contain in addition to the active ingredient, conventional excipients such as diluents, e.g. lactose, calcium phosphate, sorbitol and the like, lubricants, e.g. magnesium stearate, talc, polyethylene glycol, binding agents, e.g. polyvinylpyrrolidone, gelatin, sorbitol, tragacanth, acacia, flavoring agents, and acceptable disintegrating and wetting agents. The liquid preparations generally in the form of aqueous or oily solutions or suspensions, may contain conventional additives such as suspending agents. For topical use the compounds of the present invention may also be prepared in suitable forms to be applied to the skin, the mucous membranes of the nose and throat or bronchial tissues and may conveniently take the form of creams, ointments, liquid sprays or inhalants, lozenges, or throat paints.

For medication of the eyes or ears, the preparation may be presented in liquid or semi-liquid form formulated in hydrophobic or hydrophilic bases as ointments, creams, lotions, paints, or powders.

For rectal administration the compounds of the invention are administered in the form of suppositories admixed with conventional vehicles, such as, for example, cocoa butter, wax, spermaceti or polyethylenglycols and their derivatives.

Compositions for injection may take such forms as suspensions, solutions, or emulsions in oily or aqueous vehicles, and may contain formulatory agents such as suspending, stabilizing and/or dispersing agents.

Alternatively, the active ingredient may be in powder form for reconstitution at the time of delivery with a suitable vehicle, such as sterile water.

The amount of active principle to be administered depends on various factors such as the size and conditions of the subject to be treated, the route and frequency of administration, and the causative agent involved.

The compounds of the invention are generally effective at a dosage comprised between 0.3 and 30 mg of active ingredient per kg of body weight, preferably divided in 2 to 4 administrations per day. Particularly desirable compositions are those prepared in the form of dosage units containing from 20 to 600 mg per unit.

## EXAMPLES

### GENERAL PROCEDURES

In the following examples the starting material may be the 34-de(acetylglucosaminyl)-34-deoxy teicoplanin $A_2$ complex (i.e. a mixture of compounds obtained from teicoplanin $A_2$ complex according to the procedure of European Patent Application Publication No. 290922, a single component thereof or any mixture of two or more of said components).

The typical complex mixture essentially consists of five components corresponding to formula I above wherein the aliphatic acyl moieties of the beta-D-2-deoxy-2-aminoglycopyranosyl radical represented by the

symbol A are respectively:
(Z)-4-decenoyl ($AC_1$), 8-methylnonanoyl ($AC_2$), decanoyl ($AC_3$), 8-methyldecanoyl ($AC_4$) and 9-methyldecanoyl ($AC_5$),
B is hydrogen, M is alpha-D-mannopyranosyl and Y is OH. This mixture is identified by the acronym $TGAC_{1-5}$. When one of the single components of said mixture is employed as the starting material it is identified as follows: $TGAC_1$, $TGAC_2$, $TGAC_3$, $TGAC_4$ or $TGAC_5$, depending on the specific aliphatic acyl rest of the above mentioned aminoglucopyranosyl radical.

When a mixture of one or more components is used it is indicated according to the same system as for the complex. For instance, the acronym $TGAC_{2-5}$ indicates the mixture of the components 2 to 5 wherein component 1 is no longer present. This mixture is currently obtained from teicoplanin $A_2$ complex according to the procedure of European Patent Application Publication No. 290922 when catalytic hydrogenation is applied which saturates the double bond of component 1 transforming it into component 3. The acronym $TGAC_{2,3}$ indicates a mixture of the components 2 and 3 and the acronym $TGAC_{4,5}$ indicates a mixture of the components 4 and 5.

The resulting end products in the following table III are identified by reference to formula I above with the indication for the symbol A of the particular aliphatic acyl substituent of the beta-D-2-deoxy-2-aminoglucopyranosyl radical (A/AC) by using the conventional terms $AC_1$, $AC_2$, $AC_3$, $AC_4$, $AC_5$ as explained above. When a mixture of two or more components is obtained, this is shown through the same formal description as above.

HPLC Analysis is carried out with a Varian mod. 5000 LC pump equipped with a 20 microliter loop injector Rheodyne mod. 7125 and a UV detector at 254 nm.
Columns: pre-column (1.9 cm.) Hibar LiChro Cart 25-4 (Merck) pre-packed with Lichrosorb RP-8 (20-30 micrometer) follows by a column Hibar RT 250-4 (Merck) pre-packed with LiChrosorb RP-8 (10 micrometer). Eluents: A, 0.2% aq. $HCOONH_4$; B, $CH_3CN$. Flow rate: 2 mL/min. Infection: 20 microliter.
Elution: linear gradient from 20 to 60% of B in A in 30 min. The retention times of some representative compounds are reported in TABLE IIIb.

Acid-Base Titrations: The products are dissolved in MCS (methylcellosolve):$H_2O$ 4:1 (v/v), then an excess of 0.01 M HCl in the same solvent mixture is added and the resulting solutions are titrated with 0.01N NaOH. Equivalent weight of some representative compounds are reported in TABLE IIIa.

$^1$H-NMR spectra at 500 MHz are recorded in the temperature range from 20°C to 30°C on a Bruker AM 500 spectrometer in DMSO-$d_6$ with tetramethylsilane (TMS) as the internal reference (delta = 0.00 ppm). Table IIIc reports the most significant chemical shifts (delta, ppm) of some representative compounds.

METHOD OF PREPARATION

a) A solution of 4 g (about 2.5 mmol) of a TGAC (the complex, a single component thereof, or a mixture of two or more of the single components) and 0.36 mL (about 2.6 mmol) of triethylamine (TEA) in 20 mL of DMF is stirred at room temperature for 30 min., while adding 0.4 mL (about 2.8 mmol) of benzyl chloroformate. Then, additional 0.4 mL (about 3.3 mmol) of TEA and 4 mL (about 65 mmol) of chloroacetonitrile are added and stirring is continued at room temperature for 20 h. The reaction mixture is poured into 300 mL of ethyl acetate, the precipitated solid is collected by filtration, and washed with 100 mL of ethyl ether, yielding (after drying in vacuo at room temperature overnight) 4.3 g of crude cyanomethyl ester of $N^{15}$-carbobenzyloxy TGAC.

b) To a stirred solution of the above product in 30 mL of DMF, 35 mmol of the proper reactant amine is added, and the resulting solution is stirred at room temperature overnight. Then, 25 mL of absolute ethanol is added, followed by 250 mL of ethyl acetate. The precipitated solid is collected by filtration, washed with 100 mL of ethyl ether, and dried in vacuo at room temperature for 4 hours, yielding 4.1 g of crude $N^{15}$-carbobenzyloxy compound of formula I, which

c) is dissolved in 350 mL of a mixture methanol:0.01N HCl 7:3 (v/v). The resulting solution is adjusted at pH 3.0 with 1N HCL and hydrogenated at 1 atm and room temperature, in the presence of 4 g of 5% Pd/C, while absorbing 120 mL of hydrogen gas within 2 hours. The catalyst is filtered off and the clear filtrate is adjusted at pH 6.5 with 1N NaOH. After adding 300 mL of n-butanol and 15 g of silanized Silica-gel (0.06-0.2 mm, Merck), solvents are evaporated at 40°C under reduced pressure. The solid residue is suspended in 200 mL of water and the resulting suspension is loaded at the top of a column of 400 g of the same silanized Silica-gel in water. The column is developed with a linear gradient from 10% to 80% of acetonitrile in 0.1N acetic acid in 20 hours at the flow rate of about 250 mL/h, while collecting 25 mL fractions, which are checked by HPLC. Those fractions containing pure compounds of

the title are pooled, and the resulting solution is adjusted at pH 8.5 with 1N NaOH, and then it is concentrated at 40°C under reduced pressure to a small volume (about 50 mL). The solid which separates is collected by centrifugation, washed with 10 mL of water, then with 250 mL of ethyl ether. After drying at room temperature in vacuo overnight, the compound of the formula I is obtained, as the free base.

For the manufacture of the invention compounds where the $AC_1$ moiety is still present, the step a) is modified by reacting the $TGAC_{1-5}$ complex or the $TGAC_1$, or a mixture of two or more components containing it, with 2,2,2-trichloro-t-butoxy-chloroformate according to the same procedure as above and the first portion of step c) is replaced by contacting the resulting $C^{63}$-amide $N^{15}$-protected amine with zinc in acetic acid according to the procedure described by H. Eckert et al. in Angew. Chem. Int. Ed. Engl. 17, No.5, 361-362 (1978). The purification is carried out in the same way as described in the second part of step c).

By using the appropriate reagents TGAC and a amine of formula:

$NHR-[(CH_2)_mNR^1]_n-X-[(CH_2)_kNR^2]_h-(CH_2)_p-NR^3R^4$

under the conditions described above, the compounds represented in Table III are obtained.

## TABLE III

| Comp. | Reagents | | End product of formula I | | | |
|---|---|---|---|---|---|---|
| No. | TGAC | Amine | A/AC(*) | B | M(**) | Y |
| 1 | $TGAC_{2-5}$ | $NH_2(CH_2)_3N(CH_3)_2$ | $AC_{2-5}$ | -H | -M | $-NH(CH_2)_3N(CH_3)_2$ |
| 2 | $TGAC_{2-5}$ | $NH_2(CH_2)_3NH(CH_2)_2OH$ | $AC_{2-5}$ | -H | -M | $-NH(CH_2)_3NH(CH_2)_2OH$ |
| 3 | $TGAC_{2-5}$ | $NH_2(CH_2)_3NH(CH_2)_4NH_2$ | $AC_{2-5}$ | -H | -M | $-NH(CH_2)_3NH(CH_2)_4NH_2$ (1) |
| 4 | $TGAC_{2-5}$ | $NH_2(CH_2)_3NH(CH_2)_4NH_2$ | $AC_{2-5}$ | -H | -M | $-NH(CH_2)_4NH(CH_2)_3NH_2$ (2) |
| 5 | $TGAC_{2-5}$ | $NH_2(CH_2)_3NH(CH_2)_4NH(CH_2)_3NH_2$ | $AC_{2-5}$ | -H | -M | $-NH(CH_2)_3NH(CH_2)_4NH(CH_2)_3NH_2$ |
| 6 | $TGAC_2$ | $NH_2(CH_2)_3N(CH_3)_2$ | $AC_2$ | -H | -M | $-NH(CH_2)_3N(CH_3)_2$ |
| 7 | $TGAC_2$ | $NH_2(CH_2)_3NH(CH_2)_2OH$ | $AC_2$ | -H | -M | $-NH(CH_2)_3NH(CH_2)_2OH$ |

Continued... TABLE III

| Comp. | Reagents | | End product of formula I | | | |
|---|---|---|---|---|---|---|
| No. | TGAC | Amine | A/AC(*) | B | M(**) | Y |
| 8 | $TGAC_2$ | $NH_2(CH_2)_3NH(CH_2)_4NH_2$ | $AC_2$ | -H | -M | $-NH(CH_2)_3NH(CH_2)_4NH_2$ (1) |
| 9 | $TGAC_2$ | $NH_2(CH_2)_3NH(CH_2)_4NH_2$ | $AC_2$ | -H | -M | $-NH(CH_2)_4NH(CH_2)_3NH_2$ (2) |
| 10 | $TGAC_3$ | $NH_2(CH_2)_3NH(CH_2)_4NH_2$ | $AC_3$ | -H | -M | $-NH(CH_2)_3NH(CH_2)_4NH_2$ (1) |
| 11 | $TGAC_3$ | $NH_2(CH_2)_3NH(CH_2)_4NH_2$ | $AC_3$ | -H | -M | $-NH(CH_2)_4NH(CH_2)_3NH_2$ (2) |
| 12 | $TGAC_{2-5}$ | $NH_2(CH_2)_3NH(CH_2)_2OH$ | $AC_{2,3}$ | -H | -M | $-NH(CH_2)_3NH(CH_2)_2OH$ (3) |
| 13 | $TGAC_{2-5}$ | $NH_2(CH_2)_3NH(CH_2)_2OH$ | $AC_{4,5}$ | -H | -M | $-NH(CH_2)_3NH(CH_2)_2OH$ (3) |
| 14 | $TGAC_{2-5}$ | $NH(CH_2)_3NH(CH_2)_4NH(CH_2)_3NH_2$ | $AC_{2,3}$ | -H | -M | $-NH(CH_2)_3NH(CH_2)_4NH(CH_2)_3NH_2$ (4) |

Continued... TABLE III

| Comp. | Reagents | | End product of formula I | | | |
|---|---|---|---|---|---|---|
| No. | TGAC | Amine | A/AC(*) | B | M(**) | Y |
| 15 | $TGAC_{2-5}$ | $NH_2(CH_2)_3NH(CH_2)_4NH(CH_2)_3NH_2$ | $AC_{4,5}$ | -H | -M | $-NH(CH_2)_3NH(CH_2)_4NH(CH_2)_3NH_2$ (4) |
| 16 | $TGAC_{2-5}$ | $NH_2(CH_2)_3NH(CH_2)_4NH_2$ | $AC_{4,5}$ | -H | -M | $-NH(CH_2)_3NH(CH_2)_4NH_2$ (5) |
| 17 | $TGAC_{2-5}$ | $NH_2(CH_2)_3NH(CH_2)_4NH_2$ | $AC_{4,5}$ | -H | -M | $-NH(CH_2)_4NH(CH_2)_3NH_2$ (6) |
| 18 | $TGAC_{1-5}$ | $NH_2(CH_2)_3$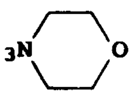 | $AC_{1-5}$ | -H | -M | $-NH(CH_2)_3$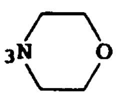 |
| 19 | $TGAC_2$ | $NH_2(CH_2)_2NH(CH_2)_2NH_2$ | $AC_2$ | -H | -M | $-NH(CH_2)_2NH(CH_2)_2NH_2$ |
| 20 | $TGAC_{2-5}$ | $NH_2(CH_2)_3NH(CH_2)_3NH_2$ | $AC_{2-5}$ | -H | -M | $-NH(CH_2)_3NH(CH_2)_3NH_2$ |
| 21 | $TGAC_3$ | $NH_2(CH_2)_3NH(CH_2)_2NH(CH_2)_3NH_2$ | $AC_3$ | -H | -M | $-NH(CH_2)_3NH(CH_2)_2NH(CH_2)_3NH_2$ |

Continued... TABLE III

| Comp. | Reagents | | End product of formula I | | | |
|---|---|---|---|---|---|---|
| No. | TGAC | Amine | A/AC(*) | B | M(**) | Y |
| 22 | $TGAC_1$ | $NH_2(CH_2)_3NH(CH_2)_3NH(CH_2)_3NH_2$ | $AC_1$ | -H | -M | $-NH(CH_2)_3NH(CH_2)_3NH(CH_2)_3NH_2$ |
| 23 | $TGAC_{1-5}$ | $NH_2(CH_2CH_2NH)_4CH_2CH_2NH_2$ | $AC_{1-5}$ | -H | -M | $-NH(CH_2CH_2NH)_4CH_2CH_2NH_2$ |
| 24 | $TGAC_{1-5}$ | $NH_2(CH_2)_3N$ 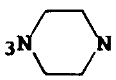 $N(CH_2)_3NH_2$ | $AC_{2-5}$ | -H | -M | $-NH(CH_2)_3N$ 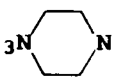 $N(CH_2)_3NH_2$ |
| 25 | $TGAC_5$ | $NH_2(CH_2)_3N$ 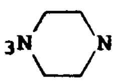 $N(CH_2)_3N(CH_3)_2$ | $AC_5$ | -H | -M | $-NH(CH_2)_3N$ 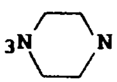 $N(CH_2)_3N(CH_3)_2$ |
| 26 | $TGAC_{4,5}$ | $NH_2(CH_2)_3N$ 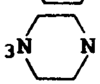 $N(CH_2)_3N(n-C_4H_9)_2$ | $AC_{4,5}$ | -H | -M | $-NH(CH_2)_3N$ 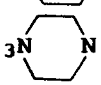 $N(CH_2)_3N(n-C_4H_9)_2$ |
| 27 | $TGAC_{2-5}$ | $NH(CH_3)(CH_2)_3NHCH_3$ | $AC_{2-5}$ | -H | -M | $-N(CH_3)(CH_2)_3NHCH_3$ |
| 28 | $TGAC_2$ | $NH(CH_3)(CH_2)_3N(CH_3)_2$ | $AC_2$ | -H | -M | $-N(CH_3)(CH_2)_3N(CH_3)_2$ |

Continued... **TABLE III**

| Comp. No. | Reagents: TGAC | Reagents: Amine | End product of formula I: A/AC(*) | B | M(**) | Y |
|---|---|---|---|---|---|---|
| 29 | $TGAC_4$ | $NH_2(CH_2)_3N$ 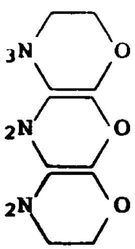 | $AC_4$ | -H | -M | $-NH(CH_2)_3N$ 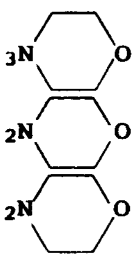 |
| 30 | $TGAC_{2-5}$ | $NH_2(CH_2)_2N$ | $AC_{2-5}$ | -H | -M | $-NH(CH_2)_2N$ |
| 31 | $TGAC_{1-5}$ | $NH_2(CH_2)_2N$ | $AC_{2-5}$ | -H | -M | $-NH(CH_2)_2N$ |
| 32 | $TGAC_{2-5}$ | $NH_2(CH_2)_3N[(CH_2)_3NH_2]_2$ | $AC_{2-5}$ | -H | -M | $-NH(CH_2)_3N[(CH_2)_3NH_2]_2$ |
| 33 | $TGAC_2$ | $NH_2(CH_2)_3N[(CH_2)_3NH_2]_2$ | $AC_2$ | -H | -M | $-NH(CH_2)_3N[(CH_2)_3NH_2]_2$ |

Legenda

(*)= $AC_1$= (Z)-4-decenoyl
$AC_2$= 8-methylnonanoyl
$AC_3$= decanoyl
$AC_4$= 8-methyldecanoyl
$AC_5$= 9-methyldecanoyl

(**)-M= alpha-D-mannopyranosyl

(1) and (2): The two products simultaneously obtained by reaction of the two different amino groups are separated during the reverse phase column chromatography.

(3): The product is obtained by carrying out the same procedure for the preparation of compound 2 but separating and pooling together only those fractions of the reverse-phase chromatography that show very close $t_R$ (min.) values when checked by HPLC (instead of pooling together all fractions which contain reaction products of the formula I).

(4): The product is obtained by carrying out the same procedure for the preparation of compound 5 but separating and pooling together only those fractions of the reverse-phase chromatography that show very close $t_R$ (min.) values when checked by HPLC (instead of pooling together all fractions which contain reaction products of the formula I).

(5): The product is obtained by carrying out the same procedure for the preparation of compound 3 but separating and pooling together only those fractions

of the reverse-phase chromatography that show very close $t_R$ (min.) values when checked by HPLC (instead of pooling together all fractions which contain reaction products of the formula I).

(6): The product is obtained by carrying out the same procedure for the preparation of compound 4 but separating and pooling together only those fractions of the reverse-phase chromatography that show very close $t_R$ (min.) values when checked by HPLC (instend of pooling together all fractions which contain reaction products of the formula I).

## TABLE IIIa

Yields and equivalent weight (EW) of some representative compounds of formula I. Between brackets are indicated the number of equivalents titrated for each molecule.

| Compound No. | Yield % | EW |
|---|---|---|
| 1 | 43 | 850 (x2) |
| 2 | 49 | 870 (x2) |
| 3 | 27 | 580 (x3) |
| 4 | 29 | 610 (x3) |
| 5 | 61 | 460 (x4) |
| 6 | 52 | 855 (x2) |
| 7 | 47 | 890 (x2) |
| 8 | 28 | 600 (x3) |
| 9 | 25 | 590 (x3) |

## Continued... TABLE IIIa

| Compound No. | Yield % | EW |
|---|---|---|
| 10 | 31 | 545 (x3) |
| 11 | 23 | 570 (x3) |
| 12 | 27 | 865 (x2) |
| 13 | 39 | 860 (x2) |
| 14 | 23 | 470 (x4) |
| 15 | 37 | 495 (x4) |
| 16 | 18 | 595 (x3) |
| 17 | 37 | 590 (x3) |
| 32 | 32 | 485 (x4) |
| 33 | 36 | 490 (x4) |

TABLE IIIb

| Retention times ($t_R$) determined as described above for some representative compounds of the invention. | |
|---|---|
| Compound No. | $t_R$ (min) |
| 6 (1) | 13.8 (*) |
| 7 (2) (12) | 13.9 (*) |
| 8 (3) | 15.7 (*) |
| 9 (4) | 15.7 (*) |
| 14 (5) | 16.6 (**) (*) |
| 13 | 14.3 (***) |
| 16 | 18.1 (***) |
| 15 | 18.5 (***) |
| 33 (32) | 16.2 (*) |

(*) This value refers also to the component 2 of the mixture reported between the brackets
(**) Value referred to the component 2 of the mixture
(***) Values referred to the component 4 of the mixture

## TABLE IIIc

Significant $^1H$-NMR assignments of some representative compounds recorded in DMSO-$d_6$ with tetramethylsilane (TMS) as internal reference (delta=0.00 ppm).

| | |
|---|---|
| Compound 1: | 2.17 ($NCH_3$); 3.15, 2.32 ($CH_2$-polyamine aliphatic chain); 2.02, 1.45, 1.13, 0.82 (aliphatic acyl chain); 4.32-6.09 (peptidic CH's); 6.32-8.62 (aromatic protons and peptidic NH's) |
| Compound 2: | 3.23, 2.95, 2.11, 1.58 ($CH_2$-polyamine aliphatic chain); 2.04, 1.45, 1.15, 0.84 (aliphatic acyl chain); 3.42 (mannose); 4.36-6.13 (peptidic CH's); 6.43-8.56 (aromatic protons and peptidic NH's) |
| Compound 3: | 3.31, 2.93, 2.11, 1.58 ($CH_2$-polyamine aliphatic chain); 2.04, 1.45, 1.15, 0.82 (aliphatic acyl chain); 4.35-5.75 (peptidic CH's); 6.42-8.42 (aromatic protons and peptidic NH's) |

Continued.... TABLE IIIc

| | |
|---|---|
| Compound 4: | 3.31, 2.93, 2.11, 1.58 ($CH_2$-polyamine aliphatic chain); 2.04, 1.45, 1.15, 0.82 (aliphatic acyl chain); 4.35-5.75 (peptidic CH's); 6.42-8.42 (aromatic protons and peptidic NH's) |
| Compound 5: | 3.29, 2.93, 2.64, 2.08, 1.71, 1.22 ($CH_2$-polyamine aliphatic chain); 2.03, 1.43, 1.18, 0.84 (aliphatic acyl chain); 4.32-6.04 (peptidic CH's); 6.28-8.62 (aromatic protons and peptidic NH's) |
| Compound 32: | 3.33, 2.82 ($CH_2$-N, polyamine); 1.65 ($CH_2$-polyamine aliphatic chain); 2.03, 1.43, 1.22, 0.83 (aliphatic acyl chain); 3.45 (mannose); 4.12-5.63 (peptidic CH's); 6.31-8.53 (aromatic protons and peptidic NH's) |

## Claims

1. Use of a teicoplanin derivative of the formula I

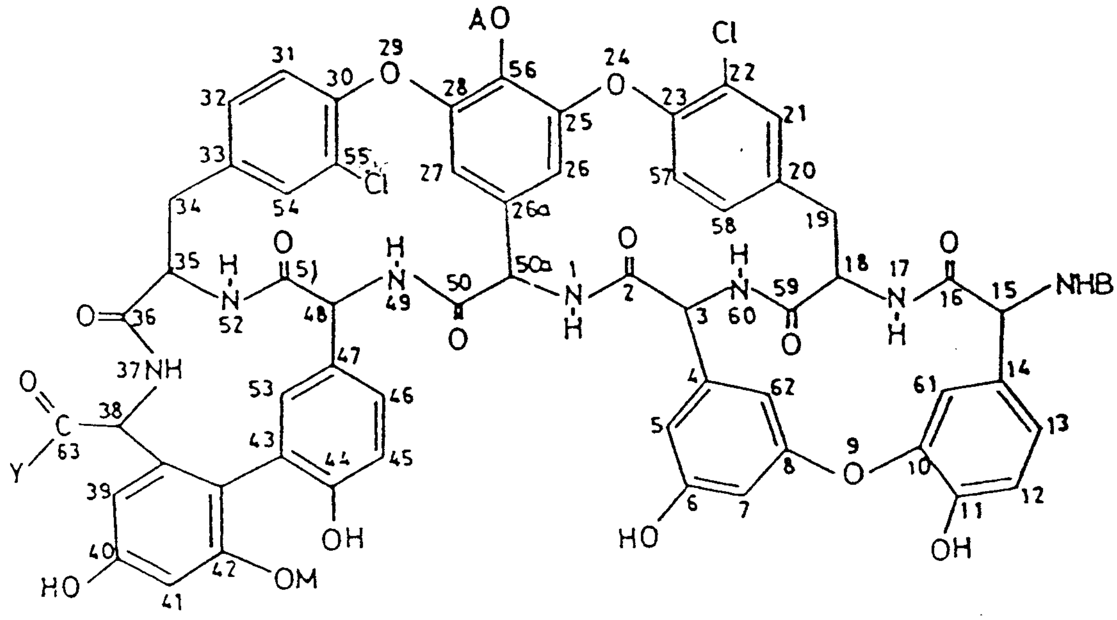

I

wherein:

A represents N[($C_9$-$C_{12}$)aliphatic acyl]-beta-D-2-deoxy-2-aminoglucopyranosyl;
B is hydrogen or a protecting group of the amine function;
M represents alpha-D-mannopyranosyl;
Y represents a di- or poly-amine group of the formula

$-NR-[(CH_2)_mNR^1]_n-X-[(CH_2)_kNR^2]_h-(CH_2)_p-NR^3R^4$

2. Use of a compound according to claim 1 wherein the ($C_9$-$C_{12}$) aliphatic acyl radical of the moiety represented by the symbol A is:
(Z)-4-decenoyl, 8-methylnonanoyl, decanoyl, 8-methyldecanoyl, 9-methyldecanoyl, 6-methyloctanoyl, nonanoyl, 10-methylundecanoyl or dodecanoyl, for the manufacture of a medicament for combatting infections caused by bacteria resistant to glycopeptide antibiotics of the vancomycin, ristocetin type including teicoplanin.

3. Use of a compound of claim 1 wherein A is as in claim 2, B is hydrogen or a protecting group of the amine function, R, $R^1$ and $R^2$, are hydrogen or linear or branched alkyl radicals of 1 to 4 carbon atoms, $R^3$ and $R^4$ are each independently hydrogen, linear or branched alkyl radicals of 1 to 4 carbon atoms optionally bearing a $NH_2$, OH or SH substituent, or $R^3$ and $R^4$ taken together with the adjacent nitrogen atom represent one of the following rings:
pyrrolidine piperidine, oxazolidine, thiazolidine, isoxazolidine, isothiazolidine, morpholine, piperazine, thiomorpholine, hexahydroazepine, hexahydro-1,5- diazepine and hexahydro-1,4-diazepine; $R^5$ is hydrogen or $C_1$-$C_4$ alkyl;

the symbols m, k and p represent integers from 2 to 6, preferably, from 2 to 4;
the symbols n and h represent 0, 1 or 2, preferably 0 or 1;
the symbol X represents a single bond or, when n is 1, taken together with the adjacent group $NR^1$ represents a bifunctional radical of the formula

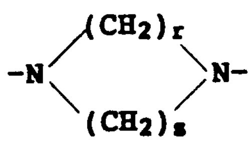

or

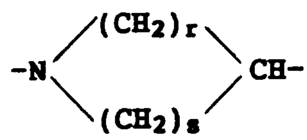

wherein r and s are both 2 or one is 1 and the other is 2 or 3;
and its addition salts with pharmaceutically acceptable acids, for the manufacture of a medicament for combatting infections caused by bacteria resistant to glycopeptide antibiotics of the vancomycin, ristocetin type including teicoplanin.

4. Use of a compound of claim 3 wherein the aliphatic acyl radical of the moiety represented by the symbol A is a ($C_{10}$-$C_{11}$) aliphatic acyl radical selected from: (Z)-4-decenoyl, 8-methylnonanoyl, decanoyl, 8-methyldecanoyl and 9-methyldecanoyl, for the manufacture of a medicament for combatting infections caused by bacteria resistant to glycopeptide antibiotics of the vancomycin, ristocetin type including teicoplanin.

5. Use of a compound of claim 4 wherein R is hydrogen or methyl, $R^1$ and $R^2$ are hydrogen, $R^3$ and $R^4$ each independently are hydrogen, a linear or branched alkyl of 1 to 4 carbon atoms optionally bearing a $NH_2$, OH or SH substituent or taken together with the adjacent nitrogen atom represents pyrrolidine, morpholine, or piperazine and $R^5$ is hydrogen or methyl;
the symbols m, k and p represent integers from 2 to 4;
the symbols n and h represent 0 or 1;
the symbol X represents a single bond or when n is 1, taken together with the adjacent group $NR^1$ represents a bifunctional radical of the formula:

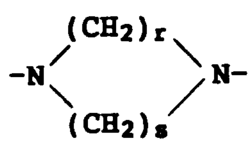

wherein r and s are both 2;
and its addition salts with pharmaceutically acceptable acids, for the manufacture of a medicament for combatting infections caused by bacteria resistant to glycopeptide antibiotics of the vancomycin, ristocetin type including teicoplanin.

6. Use of a compound of claim 1 wherein
A represents N[($C_{10}$-$C_{11}$)aliphatic acyl]-beta-D-2-deoxy-2-aminoglucopyranosyl wherein the ($C_{10}$-$C_{11}$)aliphatic acyl radical is selected from (Z)-4-decenoyl, 8-methylnonanoyl, decanoyl, 8-methyldecanoyl and 9-methyldecanoyl;
B is hydrogen or a protecting group of the amine function;
M represents alpha-D-mannopyranosyl;
Y represents a di- or poly-amine group of the formula:
$-NH(CH_2)_3N(CH_3)_2$;
$-NH(CH_2)_3NH(CH_2)_2OH$;
$-NH(CH_2)_3NH(CH_2)_4NH_2$;
$-NH(CH_2)_4NH(CH_2)_3NH_2$;
$-NH(CH_2)_3NH(CH_2)_4NH(CH_2)_3NH_2$;

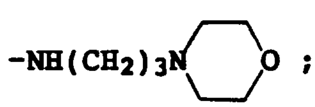

$-NH(CH_2)_2NH(CH_2)_2NH_2$;
$-NH(CH_2)_3NH(CH_2)_3NH_2$;

-NH$(CH_2)_3$NH$(CH_2)_2$NH$(CH_2)_3$NH$_2$;
-NH$(CH_2)_3$NH$(CH_2)_3$NH$(CH_2)_3$NH$_2$;
-NH$(CH_2CH_2NH)_4$CH$_2$CH$_2$NH$_2$;

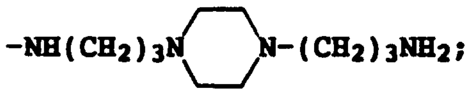

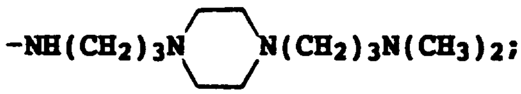

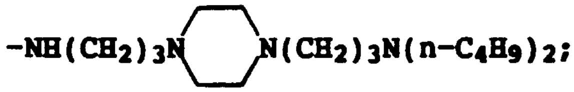

-N$(CH_3)(CH_2)_3$NHCH$_3$;
-N$(CH_3)(CH_2)_3$N$(CH_3)_2$;

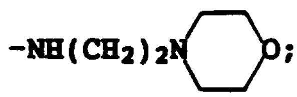

-NH$(CH_2)_3$N$[(CH_2)_3NH_2]_2$
and its addition salts with pharmaceutically acceptable acids, for the manufacture of a medicament for combatting infections caused by bacteria resistant to glycopeptide antibiotics of the vancomycin, ristocetin type including teicoplanin.

**7.** Use of a compound of claim 6 wherein Y represents
-NH$(CH_2)_3$NH$(CH_2)_4$NH$_2$
-NH$(CH_2)_3$NH$(CH_2)_4$NH$(CH_2)_3$NH$_2$ or
-NH$(CH_2)_3$N$[(CH_2)_3NH_2]_2$
and its addition salts with pharmaceutically acceptable acids, for the manufacture of a medicament for combatting infections caused by bacteria resistant to glycopeptide antibiotics of the vancomycin, ristocetin type including teicoplanin.

**8.** Use of a compound of claim 6 wherein the aliphatic acyl radical is 8-methylnonanoyl or decanoyl and Y represents
-NH$(CH_2)_3$NH$(CH_2)_4$NH$_2$
-NH$(CH_2)_3$NH$(CH_2)_4$NH$(CH_2)_3$NH$_2$ or
-NH$(CH_2)_3$N$[(CH_2)_3NH_2]_2$
and its addition salts with pharmaceutically acceptable acids, for the manufacture of a medicament for combatting infections caused by bacteria resistant to glycopeptide antibiotics of the vancomycin, ristocetin type including teicoplanin.

**9.** Use according to any of claims 1 to 8 wherein the bacteria resisistant to glycopeptide antibiotics are Enterococci.

**10.** Use according to any of claims 1 to 9 wherein the Enterococci strains resistant to glycopeptide antibiotics are selected from Enterococci faecalis and Enterococci faecium.

**Patentansprüche**

1. Verwendung eines Teicoplaninderivats der Formel I

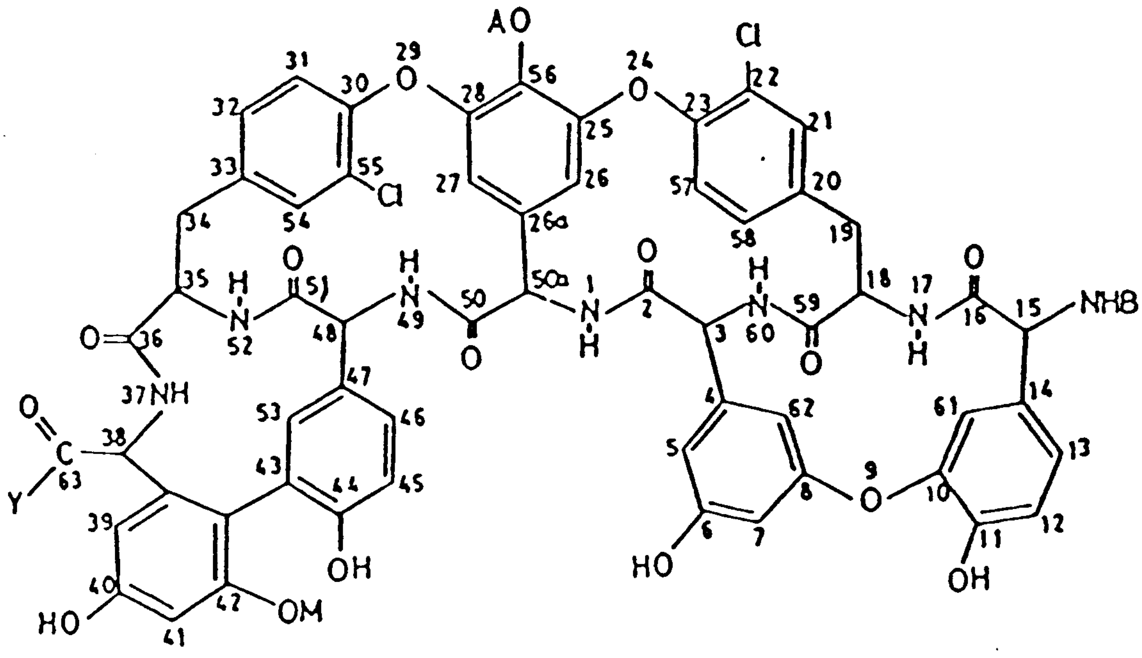

I

in der:
A einen N-[($C_9$-$C_{12}$)aliphatischen-Acyl]-beta-D-2-deoxy-2-aminoglucopyranosylrest darstellt,
B ein Wasserstoffatom oder eine Schutzgruppe der Aminfunktion ist,
M eine alpha-D-Mannopyranosylgruppe darstellt,
Y einen Di- oder Polyaminrest der Formel

$$-NR-[(CH_2)_mNR^1]_n-X-[(CH_2)_kNR^2]_h-(CH_2)_p-NR^3R^4$$

darstellt,
in der:
R ein Wasserstoffatom oder ein linearer oder verzweigter ($C_1$-$C_8$)-Alkylrest ist,
$R^1$ ein Wasserstoffatom oder ein linearer oder verzweigter ($C_1$-$C_8$)-Alkylrest ist,
$R^2$ ein Wasserstoffatom oder ein linearer oder verzweigter ($C_1$-$C_8$)-Alkylrest ist,
$R^3$ und $R^4$ jeweils unabhängig Wasserstoffatome, lineare oder verzweigte ($C_1$-$C_8$)-Alkylreste darstellen, die gegebenenfalls einen $NH_2$-, OH- oder SH-Substituenten tragen oder zusammen mit dem benachbarten Stickstoffatom einen 5- bis 7-gliedrigen gesättigten heterocyclischen Ring bilden, der ein weiteres Heteroatom, ausgewählt aus -S-, -O- und -$NR^5$- enthalten kann, wobei $R^5$ ein Wasserstoffatom, ein ($C_1$-$C_4$)-Alkylrest, eine Phenylgruppe oder ein Phenyl-($C_1$-$C_4$)-alkylrest ist,
m, k und p jeweils unabhängig eine ganze Zahl von 2 bis 8 darstellen,
n und h jeweils unabhängig eine ganze Zahl von 0 bis 4 darstellen,
X eine Einfachbindung darstellt, oder wenn n 1 ist, zusammen mit dem benachbarten Rest $NR^1$ einen bifunktionellen Rest der Formel:

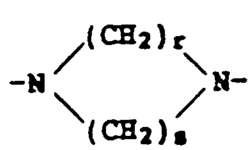 oder 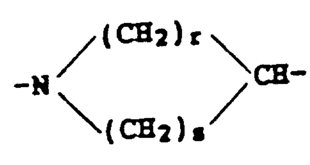

darstellt, in der r und s jeweils unabhängig eine ganze Zahl von 1 bis 6 darstellen, mit der Maßgabe, daß ihre Summe eine ganze Zahl von 3 bis 8 ist,
und seiner Additionssalze mit pharmazeutisch verträglichen Säuren zur Herstellung eines Medikaments zur Bekämpfung von Infektionen, die durch Bakterien verursacht werden, die gegenüber Glycopeptidantibiotika des Vancomycin, Ristocetintyps, einschließlich Teicoplanin, resistent sind.

2. Verwendung einer Verbindung nach Anspruch 1, in der der aliphatische ($C_9$-$C_{12}$)-Acylrest der durch das Symbol A dargestellten Einheit: eine (Z)-4-Decenoyl-, 8-Methylnonanoyl-, Decanoyl-, 8-Methyldecanoyl-, 9-Methyldecanoyl-, 6-Methyloctanoyl, Nonanoyl-, 10-Methylundecanoyl- oder Dodecanoylgruppe ist, zur Herstellung eines Medikaments zur Bekämpfung von Infektionen, die durch Bakterien verursacht werden, die gegenüber Glycopeptidantibiotika des Vancomycin, Ristocetintyps, einschließlich Teicoplanin, resistent sind.

3. Verwendung einer Verbindung nach Anspruch 1, in der A die in Anspruch 2 angegebene Bedeutung hat, B ein Wasserstoffatom oder eine Schutzgruppe der Aminfunktion ist, R, $R^1$ und $R^2$ Wasserstoffatome oder lineare oder verzweigte Alkylreste mit 1 bis 4 Kohlenstoffatomen sind, $R^3$ und $R^4$ jeweils unabhängig Wasserstoffatome, lineare oder verzweigte Alkylreste mit 1 bis 4 Kohlenstoffatomen sind, die gegebenenfalls einen $NH_2$-, OH- oder SH-Substituenten tragen, oder $R^3$ und $R^4$ zusammen mit dem benachbarten Stickstoffatom einen der folgenden Ringe darstellen:
Pyrrolidin, Piperidin, Oxazolidin, Thiazolidin, Isoxazolidin, Isothiazolidin, Morpholin, Piperazin, Thiomorpholin, Hexahydroazepin, Hexahydro-1,5-diazepin und Hexahydro-1,4-diazepin,
$R^5$ ein Wasserstoffatom oder ein $C_1$-$C_4$-Alkylrest ist,
die Symbole m, k und p ganze Zahlen von 2 bis 6, vorzugsweise von 2 bis 4, darstellen,
die Symbole n und h 0, 1 oder 2, vorzugsweise 0 oder 1, darstellen,
das Symbol X eine Einfachbindung darstellt, oder, wenn n 1 ist, zusammen mit dem benachbarten Rest $NR^1$ einen bifunktionellen Rest der Formel

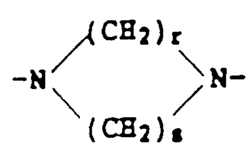

oder

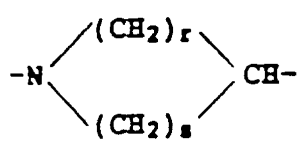

darstellt, in dem r und s beide die Zahl 2 darstellen oder eines die Zahl 1 darstellt und das andere 2 oder 3 ist,
und seiner Additionssalze mit pharmazeutisch verträglichen Säuren zur Herstellung eines Medikaments zur Bekämpfung von Infektionen, die durch Bakterien verursacht werden, die gegenüber Glycopeptidantibiotika des Vancomycin, Ristocetintyps, einschließlich Teicoplanin, resistent sind.

4. Verwendung einer Verbindung nach Anspruch 3, in der der aliphatische Acylrest der durch das Symbol A dargestellten Einheit ein aliphatischer ($C_{10}$-$C_{11}$)-Acylrest, ausgewählt aus: (Z)-4-Decenoyl-, 8-Methylnonanoyl-, Decanoyl-, 8-Methyldecanoyl und 9-Methyldecanoylgruppen ist, zur Herstellung eines Medikaments zur Bekämpfung von Infektionen, die durch Bakterien verursacht werden, die gegenüber Glycopeptidantibiotika des Vancomycin, Ristocetintyps, einschließlich Teicoplanin, resistent sind.

5. Verwendung einer Verbindung nach Anspruch 4, in der R ein Wasserstoffatom oder eine Methylgruppe ist, $R^1$ und $R^2$ Wasserstoffatome sind, $R^3$ und $R^4$ jeweils unabhängig ein Wasserstoffatom, einen linearen oder verzweigten Alkylrest mit 1 bis 4 Kohlenstoffatomen darstellen, der gegebenenfalls einen $NH_2$-, OH- oder SH-Substituenten trägt oder zusammen mit dem benachbarten Stickstoffatom eine Pyrrolidin-, Morpholin- oder Piperazingruppe darstellt, und $R^5$ ein Wasserstoffatom oder eine Methylgruppe ist,
die Symbole m, k und p ganze Zahlen von 2 bis 4 darstellen,
die Symbole n und h 0 oder 1 darstellen,
das Symbol X eine Einfachbindung darstellt, oder, wenn n 1 ist, zusammen mit dem benachbarten Rest $NR^1$ einen bifunktionellen Rest der Formel:

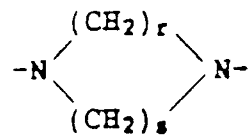

darstellt, in der r und s 2 sind,
und seiner Additionssalze mit pharmazeutisch verträglichen Säuren zur Herstellung eines Medikaments zur Bekämpfung von Infektionen, die durch Bakterien verursacht werden, die gegenüber Glycopeptidantibiotika des Vancomycin, Ristocetintyps, einschließlich Teicoplanin, resistent sind.

**6.** Verwendung einer Verbindung nach Anspruch 1, in der
A einen N-[($C_{10}$-$c_{11}$)aliphatischen-Acyl]-beta-D-2-deoxy-2-aminoglucopyranosylrest darstellt, in dem der aliphatische ($C_{10}$-$C_{11}$)-Acylrest ausgewählt ist aus (Z)-4-decenoyl-, 8-Methylnonanoyl, Decanoyl-, 8-Methyldecanoyl- und 9-Methyldecanoylgruppen,
B ein Wasserstoffatom oder eine Schutzgruppe der Aminfunktion ist,
M eine alpha-D-Mannopyranosylgruppe darstellt,
Y einen Di- oder Polyaminrest der Formel:
$-NH(CH_2)_3N(CH_3)_2$;
$-NH(CH_2)_3NH(CH_2)_2OH$;
$-NH(CH_2)_3NH(CH_2)_4NH_2$;
$-NH(CH_2)_4NH(CH_2)_3NH_2$;
$-NH(CH_2)_3NH(CH_2)_4NH(CH_2)_3NH_2$;

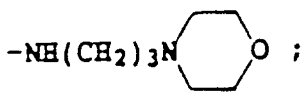

$-NH(CH_2)_2NH(CH_2)_2NH_2$;
$-NH(CH_2)_3NH(CH_2)_3NH_2$;
$-NH(CH_2)_3NH(CH_2)_2NH(CH_2)_3NH_2$;
$-NH(CH_2)_3NH(CH_2)_3NH(CH_2)_3NH_2$;
$-NH(CH_2CH_2NH)_4CH_2CH_2NH_2$;

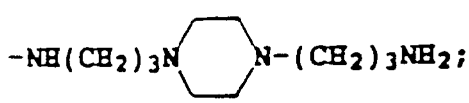

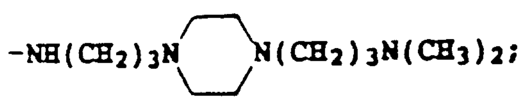

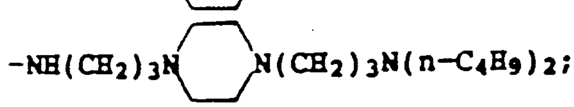

$-N(CH_3)(CH_2)_3NHCH_3$;
$-N(CH_3)(CH_2)_3N(CH_3)_2$;

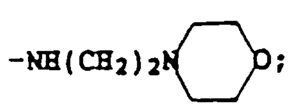

$-NH(CH_2)_3N[(CH_2)_3NH_2]_2$
darstellt,
und seiner Additionssalze mit pharmazeutisch verträglichen Säuren zur Herstellung eines Medikaments

zur Bekämpfung von Infektionen, die durch Bakterien verursacht werden, die gegenüber Glycopeptidantibiotika des Vancomycin, Ristocetintyps, einschließlich Teicoplanin, resistent sind.

**7.** Verwendung einer Verbindung nach Anspruch 6, in der Y
$-NH(CH_2)_3NH(CH_2)_4NH_2$
$-NH(CH_2)_3NH(CH_2)_4NH(CH_2)_3NH_2$ oder
$-NH(CH_2)_3N[(CH_2)_3NH_2]_2$
darstellt, und seiner Additionssalze mit pharmazeutisch verträglichen Säuren zur Herstellung eines Medikaments zur Bekämpfung von Infektionen, die durch Bakterien verursacht werden, die gegenüber Glycopeptidantibiotika des Vancomycin, Ristocetintyps, einschließlich Teicoplanin, resistent sind.

**8.** Verwendung einer Verbindung nach Anspruch 6, in der der aliphatische Acylrest eine 8-Methylnonanoyl- oder Decanoylgruppe ist und Y
$-NH(CH_2)_3NH(CH_2)_4NH_2$
$-NH(CH_2)_3NH(CH_2)_4NH(CH_2)_3NH_2$ oder
$-NH(CH_2)_3N[(CH_2)_3NH_2]_2$
darstellt, und seiner Additionssalze mit pharmazeutisch verträglichen Säuren zur Herstellung eines Medikaments zur Bekämpfung von Infektionen, die durch Bakterien verursacht werden, die gegenüber Glycopeptidantibiotika des Vancomycin, Ristocetintyps, einschließlich Teicoplanin, resistent sind.

**9.** Verwendung nach einem der Ansprüche 1 bis 8, wobei die gegen Glycopeptidantibiotika resistenten Bakterien Enterococcen sind.

**10.** Verwendung nach einem der Ansprüche 1 bis 9, wobei die gegen Glycopeptidantibiotika resistenten Enterococcen-Stämme ausgewählt sind aus Enterococci faecalis und Enterococci faecium.

## Revendications

**1.** Utilisation d'un dérivé de teicoplanine de formule

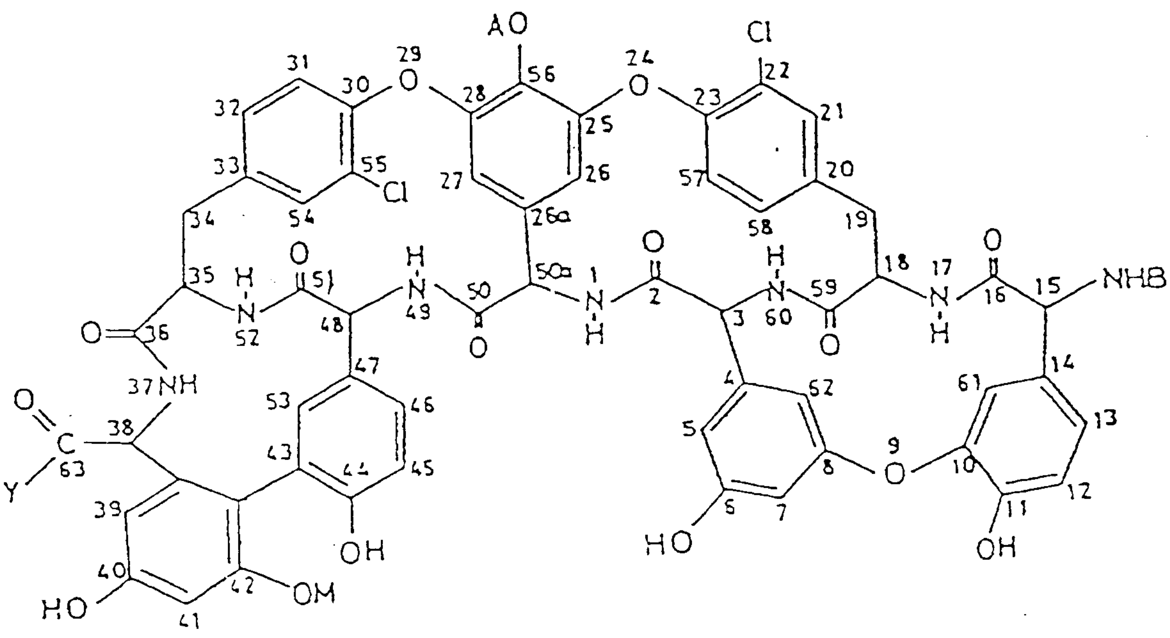

I

dans laquelle

A représente un groupememt N-[acyl ($C_9$-$C_{12}$) aliphatique]-$\beta$-D-2-désoxy-2-aminoglucopyrannosyle,

B est un atome d'hydrogène ou un groupe protecteur de la fonction amine;

M représente le groupe $\alpha$-D-mannopyrannosyle,

Y représente un groupe di- ou polyamino de formule

$-NR-[(CH_2)_mNR^1]_n-X-[(CH_2)_kNR^2]_h-(CH_2)_p-NR^3R^4$

dans laquelle

- R est un atome d'hydrogène ou un groupe alkyle en $C_1$-$C_8$ linéaire ou ramifié;
- $R^1$ est un atome d'hydrogène ou un groupe alkyle en $C_1$-$C_8$ linéaire ou ramifié;
- $R^2$ est un atome d'hydrogène ou un groupe alkyle en $C_1$-$C_8$ linéaire ou ramifié;
- $R^3$ et $R^4$ sont, indépendamment l'un de l'autre, un atome d'hydrogène ou un groupe alkyle en $C_1$-$C_8$ linéaire ou ramifié, portant éventuellement un substituant $NH_2$, OH ou SH, ou pris ensemble, avec l'atome d'azote contigu, forment un cycle hétérocyclique saturé à 5-7 chaînons pouvant contenir un autre hétéroatome choisi parmi -S-, -O- et $-NR^5-$, dans lequel $R^5$ étant un atome d'hydrogène ou un groupe alkyle en $C_1$-$C_4$, phényle ou phénylalkyle en $C_1$-$C_4$;
- m, k et p représentent, indépendamment les uns des autres, un nombre entier allant de 2 à 8;
- n et h représentent, indépendamment l'un de l'autre, un nombre entier allant de 0 à 4;
- X représente une simple liaison, ou, lorsque n est 1, peut représenter, conjointement avec le groupe contigu $NR^1$, un radical bifonctionnel de formule

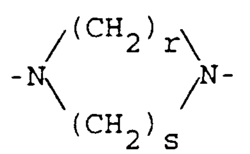 ou 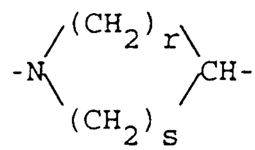

r et s représentant, indépendamment l'un de l'autre, un nombre entier allant de 1 à 6, étant entendu que leur somme est un nombre entier allant de 3 à 8;

et de leurs sels d'addition avec des acides pharmaceutiquement acceptables, pour la fabrication d'un médicament destiné à lutter contre des infections provoquées par des bactéries résistantes à des antibiotiques glycopeptidiques du type de la vancomycine et de la ristocétine, y compris la teicoplanine.

**2.** Utilisation d'un composé selon la revendication 1, dans lequel le radical acyle aliphatique en $C_9$-$C_{12}$ du fragment représenté par le symbole A est: le groupe (Z)-4-décénoyle, 8-méthylnonanoyle, décanoyle, 8-méthyldécanoyle, 9-méthyldécanoyle, 6-méthyloctanoyle, nonanoyle, 10-méthylundécanoyle ou dodécanoyle, pour la fabrication d'un médicament destiné à lutter contre des infections provoquées par des bactéries résistantes à des antibiotiques glycopeptidiques du type de la vancomycine et de la ristocétine, y compris la teicoplanine.

**3.** Utilisation d'un composé selon la revendication 1, dans lequel A est tel que dans la revendication 2, B est un atome d'hydrogène ou un groupe protecteur de la fonction amine, R, $R^1$ et $R^2$ sont des atomes d'hydrogène ou des radicaux alkyle linéaires ou ramifiés ayant de 1 à 4 atomes de carbone, $R^3$ et $R^4$ sont, indépendamment l'un de l'autre, un atome d'hydrogène ou un radical alkyle linéaire ou ramifié ayant de 1 à 4 atomes de carbone, portant éventuellement un substituant $NH_2$, OH ou SH, ou $R^3$ et $R^4$ pris ensemble, avec l'atome d'azote contigu, représentent l'un des cycles suivants:
pyrrolidine, pipéridine, oxazolidine, thiazolidine, isoxazolidine, isothiazolidine, morpholine, pipérazine, thiomorpholine, hexahydroazépine, hexahydro-1,5-diazépine et hexahydro-1,4-diazépine; $R^5$ est un atome d'hydrogène ou un groupe alkyle en $C_1$-$C_4$;

les symboles m, k et p représentent des nombres entiers allant de 2 à 6, de préférence de 2 à 4;

les symboles n et h représentent 0, 1 ou 2, de préférence 0 ou 1;

le symbole X représente une simple liaison, ou, lorsque n est 1, pris ensemble avec le groupe contigu $NR^1$, représente un radical bifonctionnel de formule

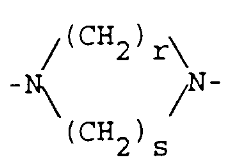 ou 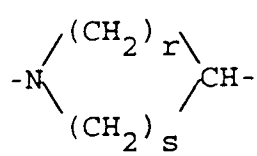

r et s étant l'un et l'autre 2 ou l'un étant 1 et l'autre étant 2 ou 3;
et de ses sels d'addition avec des acides pharmaceutiquement acceptables, pour la fabrication d'un médicament destiné à lutter contre des infections provoquées par des bactéries résistantes à des antibiotiques glycopeptidiques du type de la vancomycine et de la ristocétine, y compris la teicoplanine.

**4.** Utilisation d'un composé selon la revendication 3, dans lequel le radical acyle aliphatique du fragment représenté par le symbole A est un radical acyle aliphatique en $C_{10}$-$C_{11}$ choisi parmi les radicaux (Z)-4-décénoyle, 8-méthylnonanoyle, décanoyle, 8-méthyldécanoyle et 9-méthyldécanoyle, pour la fabrication d'un médicament destiné à lutter contre des infections provoquées par des bactéries résistantes à des antibiotiques glycopeptidiques du type de la vancomycine et de la ristocétine, y compris la teicoplanine.

**5.** Utilisation d'un composé selon la revendication 4, dans lequel R est un atome d'hydrogène ou le groupe méthyle, $R^1$ et $R^2$ sont des atomes d'hydrogène, $R^3$ et $R^4$ sont, indépendamment l'un de l'autre, un atome d'hydrogène ou un groupe alkyle linéaire ou ramifié ayant de 1 à 4 atomes de carbone, portant éventuellement un substituant $NH_2$, OH ou SH, ou pris ensemble avec l'atome d'azote contigu, représentent un cycle pyrrolidine, morpholine ou pipérazine, et $R^5$ est un atome d'hydrogène ou le groupe méthyle;

les symboles m, k et p représentent des nombres entiers allant de 2 à 4;

les symboles n et h représentent 0 ou 1;

le symbole X représente une simple liaison, ou, lorsque n est 1, pris ensemble avec le groupe contigu $NR^1$, représente un radical bifonctionnel de formule

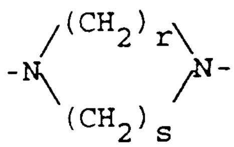

dans laquelle r et s sont l'un et l'autre 2;
et de ses sels d'addition avec des acides pharmaceutiquement acceptables, pour la fabrication d'un médicament destiné à lutter contre des infections provoquées par des bactéries résistantes à des antibiotiques glycopeptidiques du type de la vancomycine et de la ristocétine, y compris la teicoplanine.

**6.** Utilisation d'un composé selon la revendication 1, dans lequel

- A représente un groupement N-[acyl($C_{10}$-$C_{11}$) aliphatique]-$\beta$-D-2-désoxy-2-aminoglucopyrannosyle dans lequel le radical acyle aliphatique en $C_{10}$-$C_{11}$ est choisi parmi les radicaux (Z)-4-décénoyle, 8-méthylnonanoyle, décanoyle, 8-méthyldécanoyle et 9-méthyldécanoyle;
- B est un atome d'hydrogène ou un groupe protecteur de la fonction amine;
- M représente le radical $\alpha$-D-mannopyrannosyle;
- Y représente un groupe di- ou polyamino de formules:

$-NH(CH_2)_3N(CH_3)_2$;
$-NH(CH_2)_3NH(CH_2)_2OH$;
$-NH(CH_2)_3NH(CH_2)_4NH_2$;
$-NH(CH_2)_4NH(CH_2)_3NH_2$;
$-NH(CH_2)_3NH(CH_2)_4NH(CH_2)_3NH_2$;

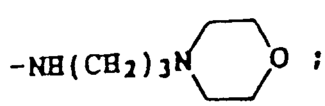

$-NH(CH_2)_2NH(CH_2)_2NH_2$;
$-NH(CH_2)_3NH(CH_2)_3NH_2$;
$-NH(CH_2)_3NH(CH_2)_2NH(CH_2)_3NH_2$;
$-NH(CH_2)_3NH(CH_2)_3NH(CH_2)_3NH_2$;

$-NH(CH_2CH_2NH)_4CH_2CH_2NH_2$;

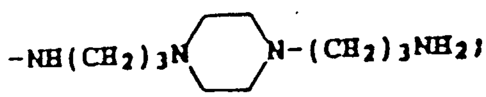

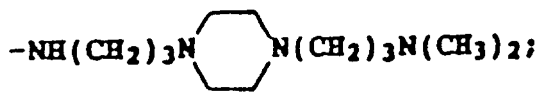

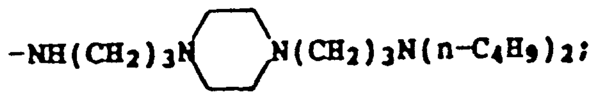

$-N(CH_3)(CH_2)_3NHCH_3$;
$-N(CH_3)(CH_2)_3N(CH_3)_2$;

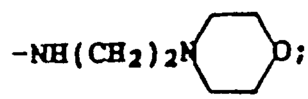

$-NH(CH_2)_3N[(CH_2)_3NH_2]_2$
et de ses sels d'addition avec des acides pharmaceutiquement acceptables, pour la fabrication d'un médicament destiné à lutter contre des infections provoquées par des bactéries résistantes à des antibiotiques glycopeptidiques du type de la vancomycine et de la ristocétine, y compris la teicoplanine.

**7.** Utilisation d'un composé selon la revendication 6, dans lequel Y représente le groupe
$-NH(CH_2)_3NH(CH_2)_4NH_2$,
$-NH(CH_2)_3NH(CH_2)_4NH(CH_2)_3NH_2$ ou
$-NH(CH_2)_3N[(CH_2)_3NH_2]_2$
et de ses sels d'addition avec des acides pharmaceutiquement acceptables, pour la fabrication d'un médicament destiné à lutter contre des infections provoquées par des bactéries résistantes à des antibiotiques glycopeptidiques du type de la vancomycine et de la ristocétine, y compris la teicoplanine.

**8.** Utilisation d'un composé selon la revendication 6, dans lequel le radical acyle aliphatique est le radical 8-méthylnonanoyle ou décanoyle, et Y représente le groupe
$-NH(CH_2)_3NH(CH_2)_4NH_2$,
$-NH(CH_2)_3NH(CH_2)_4NH(CH_2)_3NH_2$ ou
$-NH(CH_2)_3N[(CH_2)_3NH_2]_2$
et de ses sels d'addition avec des acides pharmaceutiquement acceptables, pour la fabrication d'un médicament destiné à lutter contre des infections provoquées par des bactéries résistantes à des antibiotiques glycopeptidiques du type de la vancomycine et de la ristocétine, y compris la teicoplanine.

**9.** Utilisation selon l'une quelconque des revendication 1 à 8, dans laquelle les bactéries résistantes à des antibiotiques glycopeptidiques sont des entérocoques.

**10.** Utilisation selon l'une quelconque des revendication 1 à 9, dans laquelle les souches d'entérocoques résistantes à des antibiotiques glycopeptidiques sont choisies parmi *Enterococci faecalis* et *Enterococci faecium.*